# FASCICULE DE BREVET EUROPEEN

(11) **EP 0 647 624 B1**
(45) Date de publication et mention de la délivrance du brevet: **13.08.1997**
(21) Numéro de dépôt: 94402225.0
(22) Date de dépôt: 05.10.1994
(51) Int. Cl.: C07C 403/14, C07C 403/16, C07C 403/20, C07C 403/08, C07C 403/10

(54) **Nouveaux intermédiaires de préparation de la vitamine A et des caroténoides et leur procédé de préparation**
Zwischenprodukte zur Herstellung von Vitamin A und von Carotenoiden und Verfahren zu deren Herstellung
Intermediates for the preparation of vitamin A and carotenoids and process for their preparation

(30) Priorité: 07.10.1993 FR 9311944
(43) Date de publication de la demande: 12.04.1995
(73) Titulaire: RHONE-POULENC NUTRITION ANIMALE, 92160 Antony (FR)
(72) Inventeur: Bienayme, Hugues, F-69002 Lyon (FR); Meilland, Pierre, F-69630 Chaponost (FR)
(74) Mandataire: Le Pennec, Magali

(56) Documents cités:
- CH-A- 373 373
- US-A- 3 632 860

## Description

La présente invention concerne de nouveaux intermédiaires de la vitamine A et des caroténoïdes. Elle concerne plus particulièrement des cétones alléniques ainsi que leurs intermédiaires de préparation et leur procédé d'obtention.

Il est connu selon l'article de G. SAUCY, R. MARBET paru dans Helvetica Chemica Acta, Vol. 50, pages 1158-1167, 1967 de faire une réaction de Claisen sur un alcool propargylique pour créer les enchaînements terpénique suivants :

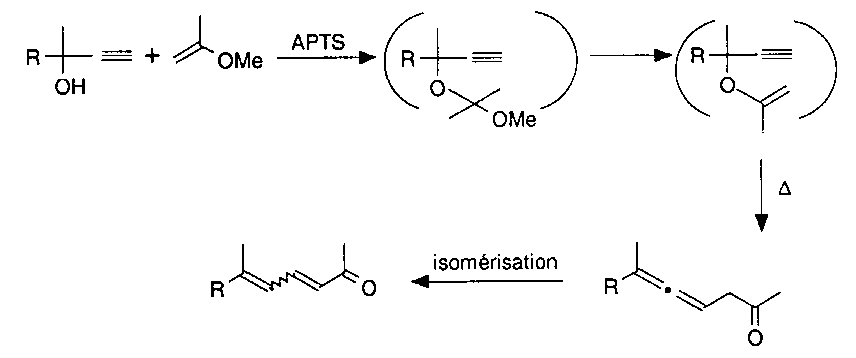

Cependant, lorsque l'alcool est à la fois propargylique et allylique c'est-à-dire lorsque R représente un résidu vinylique, le réarrangement de Claisen se fait préférentiellement du côté allylique et avec un rendement médiocre comme démontré dans la publication de Zakarova, Micropol'skya Yurkina, Filippova, Kustanovich et Samokhualov parue dans Zj. Org. Khim. 7, 1137 (1971).

Ainsi, lorsque l'on met en présence d'un acide le 3-méthyl pentène-4yne-3ol et le méthoxy-2 propène, on obtient l'acétal correspondant selon : 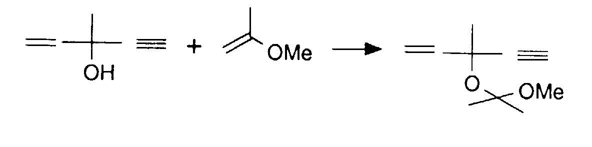 qui par chauffage en présence d'acide paratoluène sulfonique donne avec un faible rendement (30 %) un mélange des deux cétones suivantes suivant un rapport pondéral V/VI de 6/1

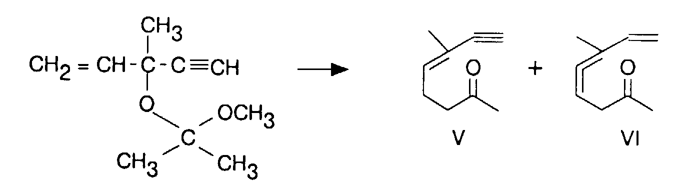

La cétone VI est très instable et par isomérie est rapidement transformée en cétone triénique dont toutes les doubles liaisons sont conjuguées. Cette cétone est ainsi obtenue avec un rendement approximatif de 4 %.

Il est donc clair que l'on ne peut pas utliser l'éthynyl-β-ionol comme matière première pour préparer par réarrangement de Claisen divers intermédiaires terpéniques tels que la cétone C₁₈.

La présente invention a permis de préparer des cétones alléniques directement utilisables dans la synthèse de la vitamine A ou des caroténoïdes avec un rendement et une sélectivité améliorés par rapport à cette publication.

La présente invention concerne des intermédiaires de la vitamine A et des caroténoïdes qui répondent à la formule (II) suivante : 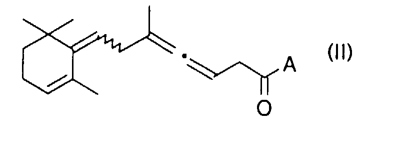 dans laquelle le motif A représente l'hydrogène, un groupe alkyle, alkényle ou un groupe alkoxyle contenant 1 à 4 atomes de carbone.

Ce dérivé est obtenu par traitement thermique d'un composé de formule (III) : 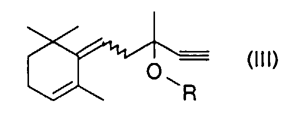 dans lequel le groupe R représente un motif alkényle linéaire ou ramifié contenant 2 à 15 atomes de carbone ou un groupe alkylcarbonyle, alkoxyalkyle, alkoxyalkényle contenant chacun 2 à 15 atomes de carbone.

On peut citer parmi les groupes alkényles les motifs suivants : 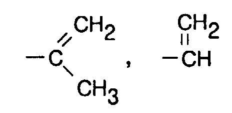 parmi les groupes alkyle carbonyle, les motifs : 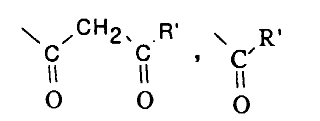 où R' représente un groupe alkyle contenant 1 à 4 atomes de carbone parmi les groupes alkoxyalkyle et alkoxyalkényle, les motifs suivants : 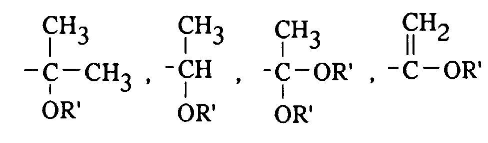 où R' représente un groupe alkyle contenant 1 à 4 atomes de carbone ou le groupe :

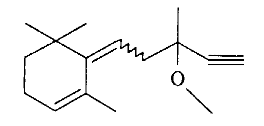

Le dérivé de formule (II) peut aussi être obtenu directement par condensation de l'éthynyl rétroαionol (III, R = H) avec un 2-alkoxypropène, de préférence le méthoxy propène, l'éthylvinyléther, l'acétate de vinyle, un acétoacétate d'alkyle, le dicétène ou un orthoacétate d'alkyle en présence d'un acide en chauffant de préférence entre 100 et 200°C.

Les intermédiaires de formule (III) : 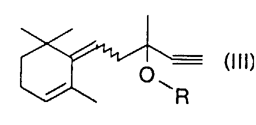 dans lesquels R représente l'hydrogène, un groupe alkyle ayant 1 à 10 atomes de carbone, un groupe alkényle ayant 2 à 10 atomes de carbone, alkylcarbonyle, alkényl carbonyle, alkoxyalkyle, alkoxyalkényle dans lesquels les groupes alkyles ont 1 à 10 atomes de carbone, les groupes alkényles ont 2 à 10 atomes de carbone, ces motifs alkyle ou alkényle pouvant être linéaires ou ramifiés et éventuellement substitués font aussi l'objet de la présente invention.

Les intermédiaires de formule (III) quand R est différent de H sont préparés par condensation de l'éthynyl rétro α ionol avec un éther d'énol ou un acétal contenant 3 à 10 atomes de carbone ou un acétoacétate d'alkyle contenant 4 à 10 atomes de carbone ou un trialkylorthoacétate contenant 5 à 10 atomes de carbone, ou du dicétène en présence éventuellement d'un catalyseur acide.

L'éthynyl rétroαionol (R = H) est préparé par un procédé qui consiste à isomériser la β ionone en rétroαionone puis à faire une éthynylation.

La transformation de la β ionone en rétroαionone a été décrite par Van Wageningen, Van der Wielen et H. Cerfontain dans Synthesis Communications, 4(6), 325-330 en 1974. Elle consiste à mettre en présence la β ionone avec un alcoolate dans un solvant aprotique polaire.

La deuxième étape consiste à faire réagir l'acétylène sur l'alpha rétro ionone en présence d'une base choisie parmi les organomagnésiens, la soude, la potasse, les alcoolates alcalins ou alcalinoterreux. Cette base est de préférence le chlorure d'isobutyl magnésium. On obtient les deux isomères cis et trans de l'éthynyl α rétroionol.

Les composés de formule (II) peuvent être ensuite isomérisés en aldéhyde, cétone et ester conjugués intermédiaires de préparation de la vitamine A et des caroténoïdes.

La présente invention sera plus complètement décrite à l'aide des exemples suivants qui ne doivent pas être considérés comme limitatifs de l'invention.

### EXEMPLE 1 : Préparation de la rétro α ionone

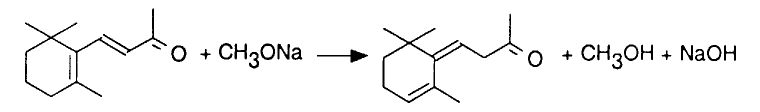

On utilise un tricol de 100 ml avec une agitation magnétique muni d'un système réfrigérant et d'un tube plongeur.

On charge 10,0 g (50,44 mmoles) de β ionone à 97 % de pureté dans 45 ml de diméthylsulfoxyde. On refroidit jusqu'à environ 15°C, on additionne alors 3,837 g de méthylate de sodium (63,9 10⁻³ mole) en enlevant le bain froid. La durée de l'addition est de 23 mn. On obtient un liquide très épais de couleur rouge. On agite encore 50 minutes entre 15 et 20°C.

On coule la masse réactionnelle sur 150 g d'eau contenant de la glace. On extrait à l'éther, lave à l'eau juqu'à neutralité. On sèche sur sulfate de magnésium.

On obtient 9,96 g de produit qui par distillation donne 7,4 g qui distille à 110°C et présente une pureté de 94%. Le rendement est de 71,2 %.

Les analyses RMN et infrarouge montrent la présence de 5% de β ionone résiduelle et les deux isomères suivants :

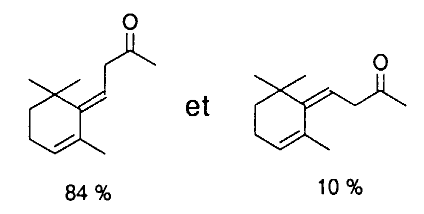

### EXEMPLE 2 : Préparation de l'éthynyl rétroαionol (composé de fomule III où R = H)

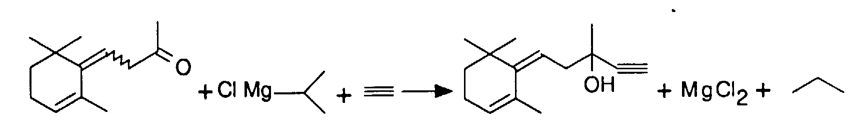

Dans un réacteur tricol de 250 ml muni d'une agitation magnétique, d'un réfrigérant, d'une arrivée de gaz et d'une ampoule de coulée on introduit 40 ml de THF à 8-10°C. On introduit ensuite l'acétylène en continu à un débit de 100 ml/mn jusqu'à saturation.

On additionne 2,31 g de chlorure d'isopropyl magnésien en 25 minutes, la température étant maintenue entre 7 et 12°C. La solution devient hétérogène, on maintient l'addition d'acétylène pendant 3 heures.

On purge à l'azote.

On coule le composé de l'essai précédent avec 10 ml de THF en 8 minutes. La solution devient limpide. On laisse réagir 1 h 30 entre 10 et 20°C. On hydrolyse la solution obtenue avec HCl 0,2 N (20 ml) vers 4 à 6°C. On reprend à l'éther, décante le chlorure de magnésium, lave plusieurs fois à l'éther puis à l'eau jusqu'à neutralité. On sèche sur sulfate de magnésium.

On obtient 5,34 g de produit brut qui est purifié sur colonne de silice avec un éluant pentane-éther éthylique. On obtient 15,9 10⁻³ mole de produit attendu (3,47 g), 2,48 10⁻³ mole de rétro αionone (0,475 g). Le rendement est d'environ 67 %.

Les analyses par RMN, infrarouge et masse ont confirmé la structure du produit attendu :

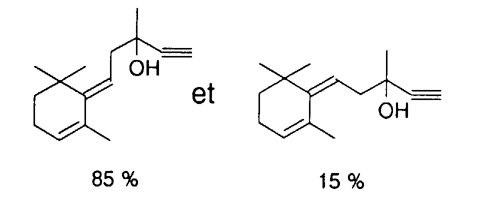

### EXEMPLE 3 : Préparation du carbonate de l'éthynyl rétroαionol

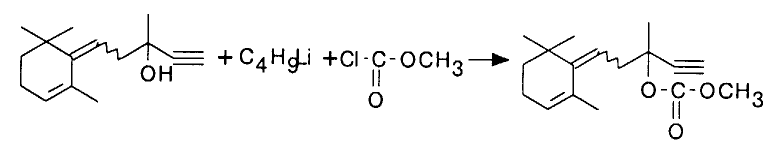

On utilise un réacteur monocol de 50 ml muni d'une agitation magnétique, d'un septum, dans un bain de carboglace.

On dissout 1,3 g du produit obtenu à l'exemple 2 dans 10 ml de THF, on refroidit à -20°C, on ajoute en 8 minutes 3,75 ml de butyllithium (6 10⁻³ mole). On refroidit à -40°C, on ajoute 910 mg de chloroformiate de méthyle en 4 minutes. On agite ensuite pendant 45 minutes à -40°C en suivant la réaction par chromatographie couche mince.

A -40°C, on ajoute 1 ml d'eau puis de l'éther. On décante et on lave à l'eau jusqu'à neutralité. On sèche sur sulfate de magnésium. On obtient 1,8 g de brut.

On purifie sur colonne de silice. On élue avec un mélange pentane/éther. On obtient 1,16 g d'un composé dont l'analyse RMN et infrarouge permettent d'identifier la présence des composés suivants :

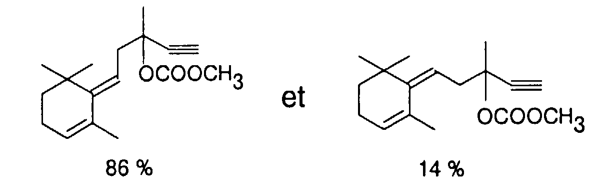

### EXEMPLE 4 : Préparation de la triméthyl 1,1,5 cyclohexène4 méthyl-9 hexène triène-6,9,10 méthylcétone (composé de structure II ; A = CH₃)

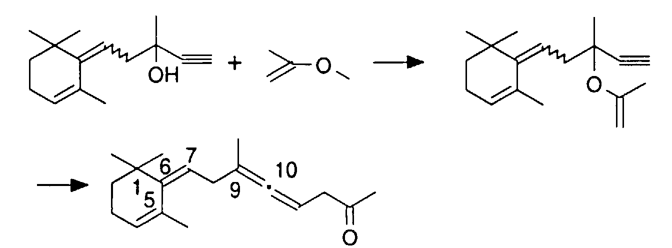

Dans un ballon tricol de 10 ml muni d'une agitation magnétique et d'un réfrigérant, on introduit 1,494 g de produit obtenu à l'exemple 2 (6,6 10⁻³ mole), 2 cristaux d'hydroquinone, on ajoute 2,4 ml de méthoxy propène (24,3 10⁻³ mole) 2 grains d'acide paratoluène sulfonique. On chauffe pendant 2 heures à 60°C puis à reflux avec un bain à 80°C pendant 4 heures. Le taux de transformation de l'éthynyl rétroαionol n'évolue plus.

On reprend le milieu réactionnel à l'éther, on lave à l'eau, puis avec une solution de carbonate acide de sodium, puis à nouveau à l'eau jusqu'à neutralité. On sèche sur sulfate de magnésium. On obtient 1,8 g d'une masse réactionnelle qui est séparée sur colonne de silice et permet la séparation de 1,27 g (4,92 10⁻³ mole) du produit recherché (rendement de 75 % par rapport à l'éthynyl rétroαionol).

Les analyses RMN, infrarouge et masse permettent de déceler la présence des deux isomères suivants :

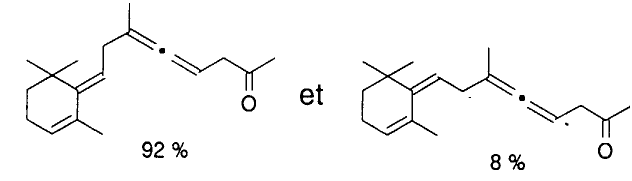

### EXEMPLE 5 COMPARATIF

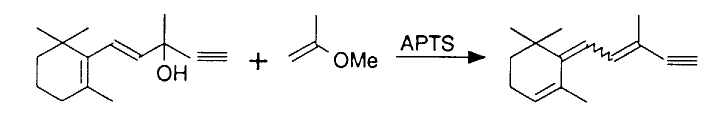

En suivant un mode opératoire identique à celui de l'exemple 4 mais en utilisant l'éthynyl-β-ionol, on ne récupère que le produit de deshydratation avec un rendement de 67 %.

### EXEMPLE 6 COMPARATIF

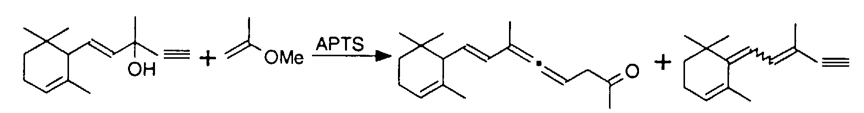

En suivant le mode opératoire de l'exemple 4 mais en utilisant l'éthynyl-α-ionol, on récupère :
- la cétone allénique attendue avec un rendement de 8,5 %
- le produit de deshydratation avec un rendement de 16,5 %

Le taux de transformation n'est que de 49 %.

### EXEMPLE 7 - Réarrangement de la cétone C₁₈ obtenue à l'exemple 4 (II, A = CH₃)

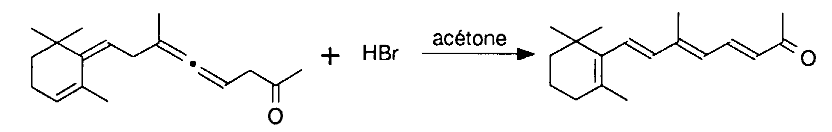

Dans un ballon, on introduit sous argon à une température comprise entre 0 et 5°C 256,6 mg (0,993 10⁻³ mole) de la cétone C₁₈ obtenue à l'exemple 4 dans 3 ml d'acétone.

On prépare une solution d'acide bromhydrique dans l'acétone (0,854 M).

On refroidit le ballon dans un bain d'eau avec de la glace et on introduit la solution d'acide bromhydrique (3 X 50 µl) en 3 heures. On obtient un taux de transformation complet en 3 heures 45 minutes.

On reprend la solution à l'éther, on lave à l'eau et au carbonate acide de sodium à froid. On sèche sur sulfate de magnésium.

On réalise une séparation sur silice avec un éluant pentane Et₂O (85/15). On obtient deux produits d'élution.

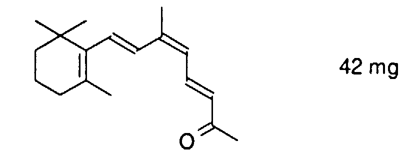

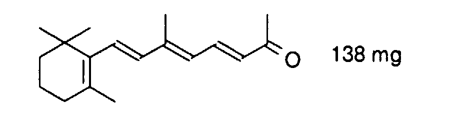

Le rendement est de 70 %.

### EXEMPLE 8 - Réarrangement de la cétone C₁₈ (formule II avec A = CH₃) en cétone C₁₈ déconjuguée.

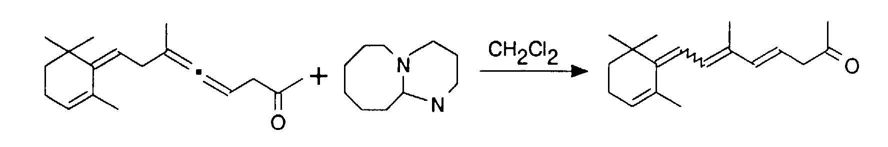

Dans un ballon, on introduit 167,2 mg (0,61 10⁻³ M) du produit obtenu à l'exemple 4 dissous dans 1 ml de chlorure de méthylène.

On ajoute 11 mg de diazabicycloundecene. En 1 heure 45 minutes la réaction est totale.

On reprend le milieu réactionnel au chlorure de méthylène, on lave à l'eau puis avec une solution d'acide chlorhydrique puis à l'eau. On obtient 162 mg de brut réactionnel.

On purifie la solution obtenue sur colonne de silice avec un éluant pentane/éther 90/10. On obtient une fraction de 88 mg avec un rendement de 53 %.

On obtient 45 % de l'isomère 9 cis et 55 % de l'isomère 9 trans.

### EXEMPLE 9 - Réarrangement partiel de la cétone C₁₈ (II, A = CH₃)

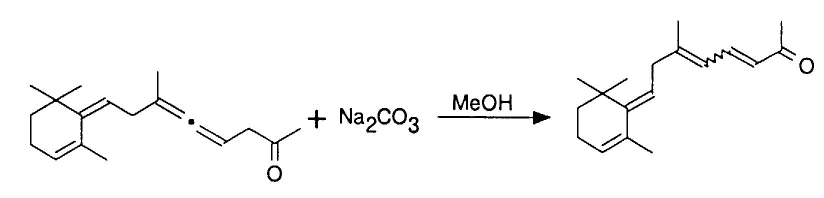

Dans un ballon, on introduit 550 mg (2,12 10⁻³ M) de produit obtenu à l'exemple 4 dissous dans 51 ml de méthanol. On maintient la température vers 20°C. On ajoute 44,4 mg de NaHCO₃. On maintient 1 heure 30 minutes à 20°C.

On concentre le méthanol sous vide. On reprend à l'éther, lave à l'eau jusqu'à neutralité, puis sèche sur MgSO₄.

On obtient 517 mg de masse réactionnelle brute.

On réalise une séparation sur colone de silice en éluant avec un mélange pentane/Et₂O (85 - 15).

On récupère 342 mg d'un mélange composé des isomères suivants :

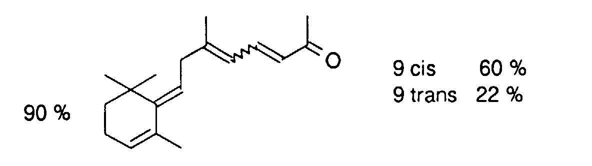

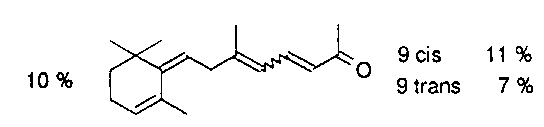

Le rendement est de 62 %.

## Revendications

1. Nouveaux intermédiaires de préparation de la vitamine A et des caroténoïdes caractérisés en ce qu'ils répondent à la formule (II) suivante : 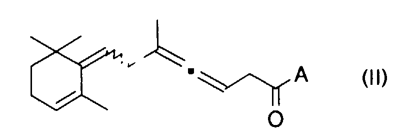 dans laquelle A représente un motif hydrogène, alkyle, alkényle ou alkoxyle contenant 1 à 4 atomes de carbone.

2. Procédé de préparation des intermédiaires selon la revendication 1 caractérisé en ce qu'on traite thermiquement un dérivé acétylénique de formule (III) suivante : 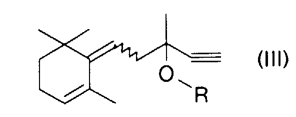 dans laquelle le groupe R représente un motif alkényle linéaire ou ramifié contenant 2 à 15 atomes de carbone ou un groupe alkylcarbonyle, alkoxyalkyle, alkoxyalkényle contenant chacun 2 à 15 atomes de carbone.

3. Procédé selon la revendication 2 caractérisé en ce que les motifs alkényles sont choisis parmi les motifs suivants :

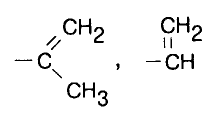

4. Procédé selon la revendication 2 caractérisé en ce que les motifs alkylcarbonyle sont choisis parmi les motifs : 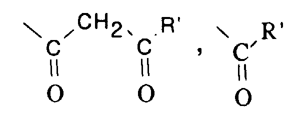 où R' représente un groupe alkyle contenant 1 à 4 atomes de carbone.

5. Procédé selon la revendication 2 caractérisé en ce que les motifs alkoxyalkyles ou alkoxyalkényles sont choisis parmi les motifs suivants : 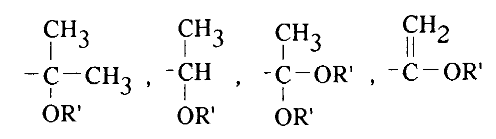 où R' représente un groupe alkyle contenant 1 à 4 atomes de carbone ou un groupe :

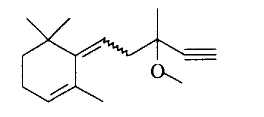

6. Procédé de préparation des intermédiaires selon la revendication 1 caractérisé en ce que l'on condense l'éthynyl rétro α ionol avec un 2-alkoxy propène en présence d'un acide.

7. Intermédiaires de préparation de composés selon la revendication 1 caractérisés en ce qu'ils répondent à la formule (III) suivante : 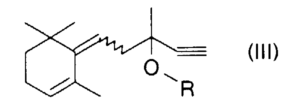 dans lesquels R représente l'hydrogène, un groupe alkyle ayant 1 à 10 atomes de carbone, un groupe alkényle ayant 2 à 10 atomes de carbone, alkylcarbonyle, alkényl carbonyle, alkoxyalkyle, alkoxyalkényle dans lesquels les groupes alkyles ont 1 à 10 atomes de carbone, les groupes alkényles ont 2 à 10 atomes de carbone, ces motifs alkyle ou alkényle pouvant être linéaires ou ramifiés et éventuellement substitués.

8. Intermédiaire selon la revendication 7 caractérisé en ce que R représente l'hydrogène.

9. Procédé de préparation d'un intermédiaire de formule (III) dans lequel R ne représente pas l'hydrogène caractérisé en qu'on fait réagir l'éthynyl rétro α ionol avec un éther d'énol ou un acétal contenant 3 à 10 atomes de carbone ou un acétoacétate d'alkyle contenant 4 à 10 atomes de carbone ou un trialkylorthoacétate contenant 5 à 10 atomes de carbone ou du dicétène en présence éventuellement d'un catalyseur acide.

10. Procédé de préparation d'un intermédiaire de formule (III) dans lequel R représente l'hydrogène caractérisé en ce que dans un première étape on met en contact la β ionone avec un alcoolate et dans une deuxième étape le composé obtenu à la première étape avec de l'acétylène en présence d'une base.

11. Procédé de préparation du rétinal caractérisé en ce qu'on traite le dérivé de formule (II) où A représente un groupe méthyle par l'acide bromhydrique.

## Claims

1. Novel intermediates for the preparation of vitamin A and carotenoids, characterized in that they correspond to formula (II) below: 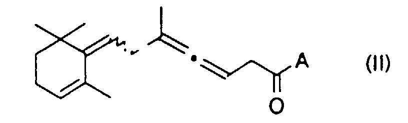 in which A represents hydrogen or an alkyl, alkenyl or alkoxy unit containing 1 to 4 carbon atoms.

2. Process for the preparation of the intermediates according to claim 1, characterized in that an acetylene derivative of formula (III) below: 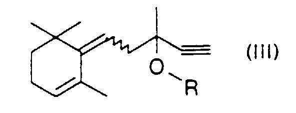 in which the group R represents a linear or branched alkenyl unit containing 2 to 15 carbon atoms or an alkylcarbonyl, alkoxyalkyl or alkoxyalkenyl group each containing 2 to 15 carbon atoms, is heat-treated.

3. Process according to claim 2, characterized in that the alkenyl units are chosen from the following units:

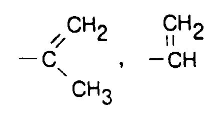

4. Process according to claim 2, characterized in that the alkylcarbonyl units are chosen from the units: 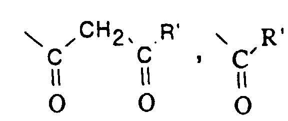 where R' represents an alkyl group containing 1 to 4 carbon atoms.

5. Process according to claim 2, characterized in that the alkoxyalkyl or alkoxyalkenyl units are chosen from the following units: 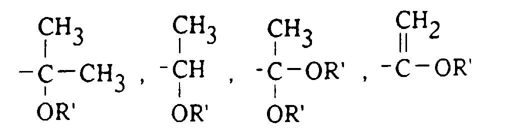 where R' represents an alkyl group containing 1 to 4 carbon atoms or a group:

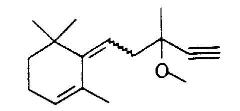

6. Process for the preparation of the intermediates according to claim 1, characterized in that ethynyl retro-α-ionol is condensed with a 2-alkoxypropene in the presence of an acid.

7. Intermediates for the preparation of compounds according to claim 1, characterized in that they correspond to formula (III) below: 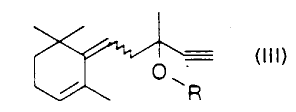 in which R represents hydrogen, an alkyl group having 1 to 10 carbon atoms, an alkenyl group having 2 to 10 carbon atoms, alkylcarbonyl, alkenylcarbonyl, alkoxyalkyl or alkoxyalkenyl in which the alkyl groups have 1 to 10 carbon atoms and the alkenyl groups have 2 to 10 carbon atoms, it being possible for these alkyl or alkenyl units to be linear or branched and optionally substituted.

8. Intermediate according to claim 7, characterized in that R represents hydrogen.

9. Process for the preparation of an intermediate of formula (III) in which R does not represent hydrogen, characterized in that ethynyl retro-α-ionol is reacted with an enol ether or an acetal containing 3 to 10 carbon atoms or an alkyl acetoacetate containing 4 to 10 carbon atoms or a trialkyl orthoacetate containing 5 to 10 carbon atoms or diketene optionally in the presence of an acid catalyst.

10. Process for the preparation of an intermediate of formula (III) in which R represents hydrogen, characterized in that, in a first step, β-ionone is placed in contact with an alkoxide and, in a second step, the compound obtained in the first step is placed in contact with acetylene in the presence of a base.

11. Process for the preparation of retinal, characterized in that the derivative of formula (II) in which A represents a methyl group is treated with hydrobromic acid.

## Patentansprüche

1. Neue Zwischenprodukte zur Herstellung von Vitamin A und Carotenoiden, dadurch **gekennzeichnet,** daß sie der folgenden Formel (II): 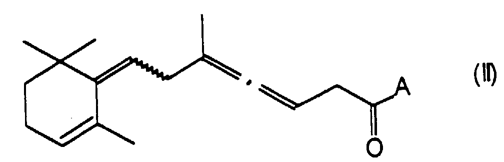 worin A ein Wasserstoffatom, einen Alkyl-, Alkenyl- oder Alkoxylrest mit 1 bis 4 Kohlenstoffatomen bedeutet, entsprechen.

2. Verfahren zur Herstellung von Zwischenprodukten nach Anspruch 1, dadurch **gekennzeichnet,** daß man ein Acetylenderivat der folgenden Formel (III): 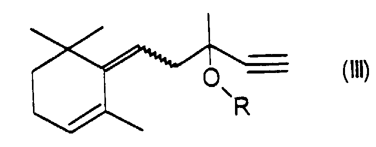 worin der Rest R einen linearen oder verzweigten Alkenylrest mit 2 bis 15 Kohlenstoffatomen oder einen Alkylcarbonyl-, Alkoxyalkyl-, Alkoxyalkenylrest mit jeweils 2 bis 15 Kohlenstoffatomen bedeutet, thermisch behandelt.

3. Verfahren nach Anspruch 2, dadurch **gekennzeichnet,** daß die Alkenylreste aus den folgenden Resten: 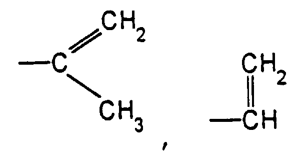 ausgewählt sind.

4. Verfahren nach Anspruch 2, dadurch **gekennzeichnet,** daß die Alkylcarbonylreste aus den Resten 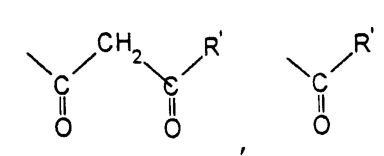 worin R' für einen Alkylrest mit 1 bis 4 Kohlenstoffatomen steht, ausgewählt sind.

5. Verfahren nach Anspruch 2, dadurch **gekennzeichnet,** daß die Alkoxyalkylreste oder Alkoxyalkenylreste aus den folgenden Resten: 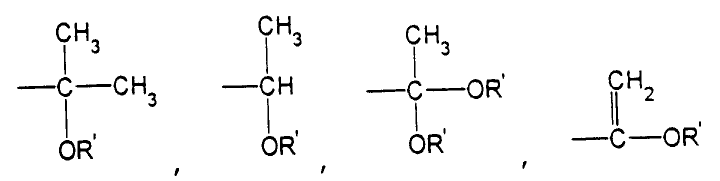 worin R' einen Alkylrest mit 1 bis 4 Kohlenstoffatomen oder eine Gruppe: 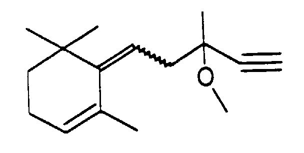 bedeutet, ausgewählt sind.

6. Verfahren zur Herstellung von Zwischenprodukten nach Anspruch 1, dadurch **gekennzeichnet,** daß man Ethinylretro-α-ionol mit einem 2-Alkoxypropen in Gegenwart einer Säure kondensiert.

7. Zwischenprodukte zur Herstellung von Verbindungen nach Anspruch 1, dadurch **gekennzeichnet,** daß sie der folgenden Formel (III): 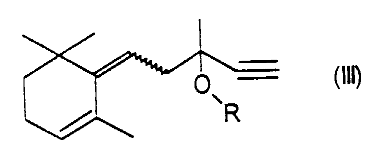 worin R Wasserstoff, einen Alkylrest mit 1 bis 10 Kohlenstoffatomen, einen Alkenylrest mit 2 bis 10 Kohlenstoffatomen, Alkylcarbonyl, Alkenylcarbonyl, Alkoxyalkyl, Alkoxyalkenyl, worin jeweils die Alkylreste 1 bis 10 Kohlenstoffatome besitzen, die Alkenylreste 2 bis 10 Kohlenstoffatome besitzen, bedeutet, entsprechen, wobei die Alkyl- oder Alkenylreste linear oder verzweigt und ggf. substituiert sein können.

8. Zwischenprodukt nach Anspruch 7, dadurch **gekennzeichnet,** daß R Wasserstoff bedeutet.

9. Verfahren zur Herstellung eines Zwischenprodukts der Formel (III), worin R nicht Wasserstoff bedeutet, dadurch **gekennzeichnet,** daß man Ethinylretro-α-ionol mit einem Enolether oder einem Acetal mit 3 bis 10 Kohlenstoffatomen oder einem Alkylacetoacetat mit 4 bis 10 Kohlenstoffatomen oder einem Trialkylorthoacetat mit 5 bis 10 Kohlenstoffatomen oder Diketen, ggf. in Gegenwart eines sauren Katalysators, umsetzt.

10. Verfahren zur Herstellung eines Zwischenprodukts der Formel (III), worin R Wasserstoff bedeutet, dadurch **gekennzeichnet,** daß man in einer ersten Stufe β-Ionon mit einem Alkoholat in Kontakt bringt und in einer zweiten Stufe die in der ersten Stufe erhaltene Verbindung mit Acetylen in Gegenwart einer Base in Kontakt bringt.

11. Verfahren zur Herstellung von Retinal, dadurch **gekennzeichnet,** daß man das Derivat der Formel (II), worin A eine Methylgruppe bedeutet, mit Bromwasserstoffsäure behandelt.
